# EUROPEAN PATENT SPECIFICATION

(11) **EP 1 801 111 B1**
(45) Date of publication and mention of the grant of the patent: **16.07.2014**
(21) Application number: 06126293.7
(22) Date of filing: 18.12.2006
(51) Int. Cl.: C07D 405/14, A61K 31/4178, A61P 9/12

(54) **Novel polymorph forms of olmesartan medoxomil**
Polymorphe Formen von Olmesartan Medoxomil
Formes polymorphes du Olmesartan Medoxomil

(30) Priority: 20.12.2005 SI 200500343
(43) Date of publication of application: 27.06.2007
(73) Proprietor: LEK Pharmaceuticals d.d., 1526 Ljubljana (SI)
(72) Inventor: Antoncic, Ljubomir, 1000, Ljubljana (SI)
(74) Representative: Prüfer & Partner GbR European Patent Attorneys

(56) References cited:
- EP-A1- 0 503 785
- WO-A-2007/017135
- KOIKE H ET AL: "OLMESARTAN MEDOXOMIL, A NOVEL POTENT ANGIOTENSIN II BLOCKER" SANKYO KENKYUSHO NEMPO - ANNUAL REPORT OF SANKYO RESEARCH LABORATORIES, TOKYO, JP, vol. 55, 2003, pages 1-89, XP002411683 ISSN: 0080-6064

## Description

### FIELD OF THE INVENTION

The present invention relates to the field of pharmaceutics, more particular to a novel form of olmesartan medoxomil, to processes for its preparation and to pharmaceutical compositions containing said form.

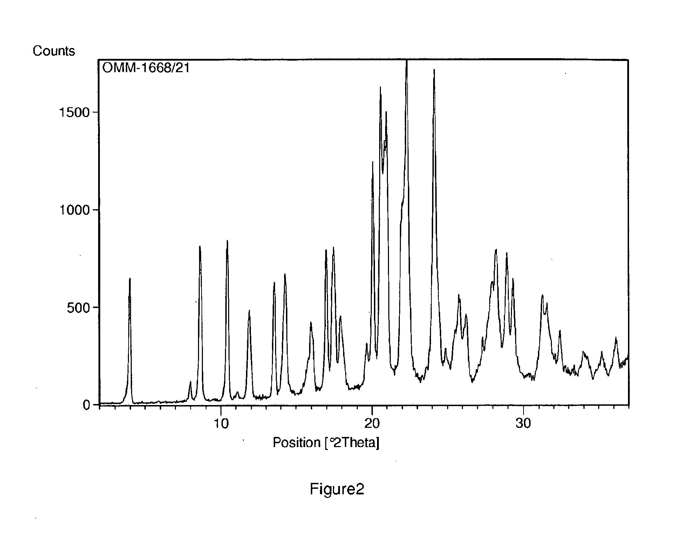

The present invention in one aspect provides for the olmesartan medoxomil hemihydrate, particularly in crystalline form, more particularly with XRPD substantially corresponding to Figure 2.

Additional aspects of the invention are the processes to prepare aforementioned hemihydrate characterized by isolation from solution in a solvent system comprising DMSO and water; by exposure of amorphous to moisture, specifically moisture is gas (specifically air, but also nitrogen) saturated with water, or air with relative humidity 60% or any range therein, more specifically air with relative humidity between 75% and 95%; at respective temperature, but preferably at 40 to 60°C.

Important aspect of the invention is the use of olmesartan medoxomil hemihydrate as a medicament and its embodiment: the pharmaceutical composition comprising olmesartan medoxomil hemihydrate and a pharmaceutically acceptable carrier.

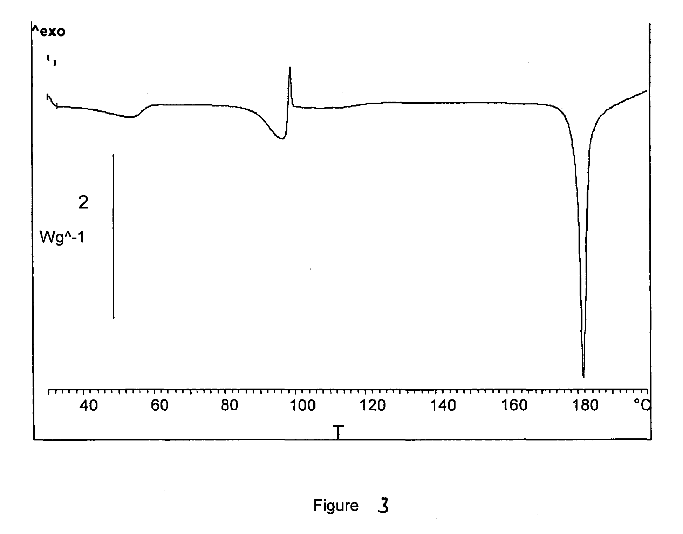

Specifically the aspects of the invention are: olmesartan medoxomil hemihydrate; characterized by that it is crystalline; further characterized by XRPD having strongest diffractions at: 20.6; 22.3; 24.2 ± 0,2° 2Theta; further characterized by XRPD having characteristic pattern exhibiting peaks at 8.7; 10.4; 17.0; 17.5; 20.1; 20.6, 20.9; 21.0; 22.3; and 24.2 ± 0,2 °2Theta; further characterized by XRPD having characteristic pattern exhibiting peaks at 4.0 (40%); 8.0 (10%); 8.7 (50%); 10.4 (50%); 11.9 (30%); 13.5 (40%); 14.3 (40%); 16.0 (20%); 17.0 (40%); 17.5 (40%); 17.9 (20%); 19.6 (10%); 20.1 (70%); 20.6 (90%); 20.9 (70%); 21.0 (80%); 22.3 (100%); 24.2 (100%); 25.8 (30%); 26.2 (20%); 27.9 (30%); 28.2 (40%); 28.9 (40%); 29.3 (30%); 31.2 (20%); 31.5 (20%); 32.4 (10%); °2 Theta, wherein values in parenthesis indicate relative rounded to nearest 10%; further characterized by XRPD having characteristic pattern substantially as in Figure 2; further characterized by melting point around 182 °C; further characterized by a DSC thermogram substantially as in Figure 3.

Invention is certain aspects a process for manufacturing olmesartan medoxomil in a hydrated form characterized by exposure of amorphous form to moisture; further wherein said olmesartan medoxomil in a hydrated form is hemihydrate; further characterized by isolation from solvent system comprising DMSO and water; further wherein the weight ratio olmesartan medoxomil : DMSO : water to be 1 : 3 - 10 : 1 - 5; further wherein the process consists of following steps: a) preparing a solution of olmesartan medoxomil in DMSO b) adding water to the solution of previous step c) crystallizing and isolating the formed olmesartan medoxomil hemihydrate; further specifically when crystallization and isolation in step c) is at room temperature, when solution of olmesartan medoxomil in step a) is DMSO optionally in mixture with another solvent and where steps a) to c) are performed at temperature from 0 to 60°C, more specifically at 10 to 50°, yet more specifically at room temperature.

Considering advantageous solubility of novel form of olmesartan, it is especially advantageous for use for preparation of immediate release pharmaceutical compositions comprising olmesartan medoxomil hemihydrate and a pharmaceutically acceptable carrier. Those compositions may be prepared by mixing the novel form of olmesartan medoxomil, i.e. hemihydrate with pharmaceutically acceptable carrier, specifically by mixing said olmesartan medoxomil with excipients selected from conventional tableting ingredients and tableting with tableting machine to prepare tablet, which may be coated.

### BACKGROUND OF THE INVENTION

Olmesartan medoxomil is an antihypertensive agent known from EP 0503785 which describes a series of 1-(biphenylmethyl)imidazole derivatives which have hypotensive activities. According to example 61 b thereof olmesartan medoxomil has a melting point 170°-172 °C.

In a review, Koike et al., "Olmesartan Medoxomil, a Novel Potent Angiotensin II Blocker" in Sankyo Kenkyusho Nenpo - Annual Report of Sankyo Research Laboratories, Tokyo, JP, vol. 55, 2003, pages 1-91 described in detail the chemistry; physicochemical properties; pharmacology; absorption, distribution, metabolism, and excretion in animals; pre-clinical safety evaluation; and clinical evaluation of olmesartan medoxomil.

Present invention discloses a new form of olmesartan medoxomil, processes for preparing this form, namely a hydrated form of olmesartan medoxomil, as well as pharmaceutical compositions containing said form.

For reference, amorphous or partially crystalline olmesartan medoxomil, processes for their preparation, use of said forms as medicament, and pharmaceutical compositions comprising said forms are described in European patent application publication EP 1 801 111 A1.

### BRIEF DESCRIPTION OF THE DRAWINGS

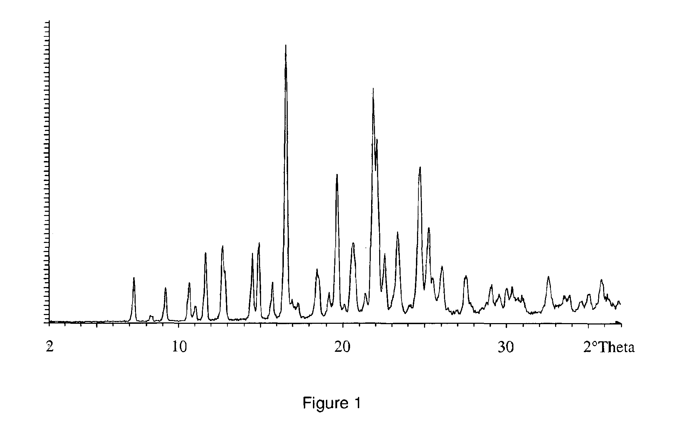

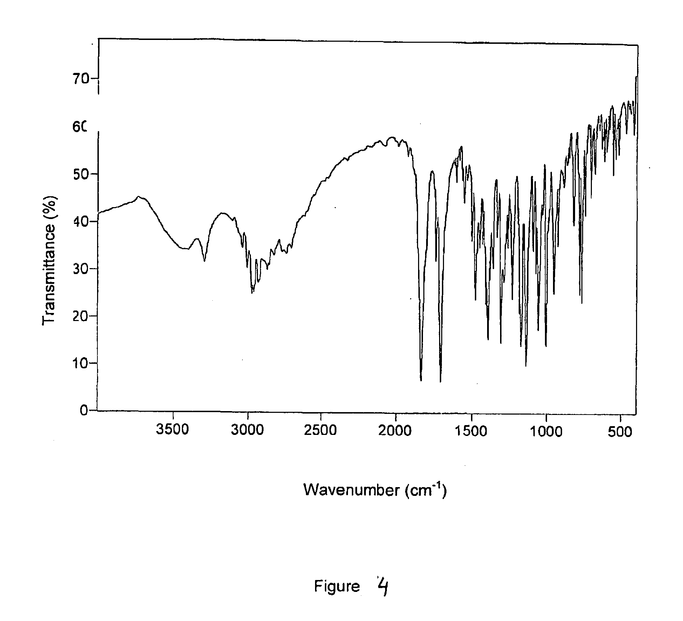

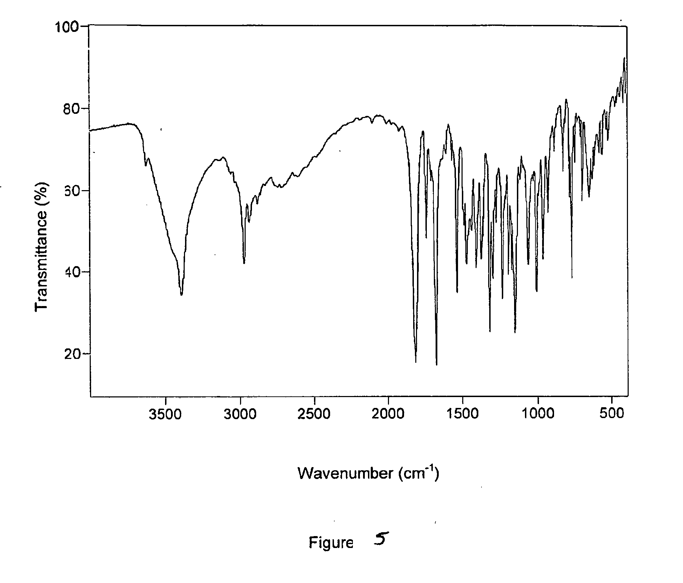

Figure 1 is an X-ray powder diffraction pattern (XRPD) of olmesartan medoxomil as produced according to EP 503785. Figure 2 is an X-ray powder diffraction pattern of olmesartan medoxomil hemihydrate. Figure 3 is a DSC thermogram of olmesartan medoxomil hemihydrate. Figure 4 is an IR pattern of olmesartan medoxomil as produced according to EP 503785. Figure 5 is an IR pattern of olmesartan medoxomil hemihydrate.

### DESCRIPTION OF THE INVENTION

Olmesartan medoxomil in a hydrated form can be isolated from the mixture of solvents such as DMSO and water. Alternatively hydrated olmesartan medoxomil can also be prepared from substantially anhydrous amorphous olmesartan medoxomil for example by exposure to moisture. Exposure to moisture can be to a gas (preferably air) saturated with water vapors, or to 75% relative humidity; specifically at temperatures between r.t. and 60°C for prolonged time, such as up to week or up to month. Hydrated olmesartan medoxomil isolated by such manner can be dried in vacuo at maximal temperature of 60 °C, preferably between 40 and 50 °C after which water content is stabilized between 1.3 - 1,8 %, preferably between 1.45 and 1,65 %, corresponding to one molecule of water per two molecules of olmesartan medoxomil. In a specific embodiment olmesartan medoxomil is a hemihydrate, having XRPD as follows: 4.0 (40%); 8.0 (10%); 8.7 (50%); 10.4 (50%); 11.9 (30%); 13.5 (40%); 14.3 (40%); 16.0 (20%); 17.0 (40%); 17.5 (40%); 17.9 (20%); 19.6 (10%); 20.1 (70%); 20.6 (90%); 20.9 (70%); 21.0 (80%); 22.3 (100%); 24.2 (100%); 25.8 (30%); 26.2 (20%); 27.9 (30%); 28.2 (40%); 28.9 (40%); 29.3 (30%); 31.2 (20%); 31.5 (20%); 32.4 (10%); °2 Theta, wherein relative intensities (given in parenthesis) are rounded to nearest 10%.

Olmesartan medoxomil hemihydrate will preferably be characterized by XRPD as above or as presented in Figure 2, specifically by diffractions at: 8.7; 10.4; 17.0; 17.5; 20.1; 20.6, 20.9; 21.0; 22.3; and 24.2 ± 0.2 °2Theta, preferably of those 20.6; 22.3; 24,2 ± 0.2 °2Theta. It can be characterized by the melting point at around 182 °C, which is sharp but can be also in the range 180 - 184 °C, as evident from Figure 3. Endotherm signal shows water removal above 80 °C, more considerably above 90 °C. The characteristic IR bands thereof, substantially as in Figure 5, are at around 1540 cm⁻¹, which is not present in prior art olmesartan medoxomil as produced according EP 503785 and at 1676, 1806, and 1818 where said prior art substance exhibits bands at 1707 and 1832 cm⁻¹.

In a process embodiment olmesartan medoxomil of any form may be dissolved at room temperature in 3 to 10 times weight excess of dimethylsulfoxide (DMSO), and to this solution water in weight ratio 1 to 5 relatively to olmesartan medoxomil is added. After up to a day (in another embodiment after 1 to 8 hours) the olmesartan medoxomil hemihydrate is isolated from the formed suspension.

The hemihydrate form exhibits transition phenomena above usual working temperatures and far above storage temperatures, therefore it is stable and suitable for preparation of pharmaceutical compositions. Moreover it exhibits advantageous properties when being used as a and/or being formulated into a pharmaceutical composition which is administered to patients. In later regard this is manifested in solubility properties in physiological fluids and within range of pH. The solubility in 1 N HCI is thus for example: of hemihydrate olmesartan medoxomil around 3 mg/ml compared to approximately 2 mg of prior art substance. Thus solubility means that when formulated into an oral pharmaceutical composition for immediate release such compositions will be easier to dissolve and will provide better bioavailability after being ingested. Immediate release compositions have no excipients or structural elements (e.g. enteric coating) which would substantially delay, prolong or condition (e.g. on pH) the release of therein contained actives.

Furthermore the new form is as soluble as prior art form in ethanol, which is a liquid commonly used in pharmaceutical technology. In further aspect of ease of manufacturing the olmesartan medoxomil hemihydrate is not hygroscopic as the prior art substance. After exposing to air moisture for 6 days, the mass of the sample of olmesartan medoxomil hemihydrate did not increase, while the mass of the prior art substance increased for 0,61 %.

### EXPERIMENTAL PART

Infrared spectra were obtained with Nicolet Nexus FTIR spectrophotometer. Samples were analyzed in KBr and scanned from 400 to 4000 cm⁻¹ with 16 scans and 2 cm⁻¹ resolution. Major differences of IR spectra of forms according to EP 503785 and form of the present invention are presented in Figures 4 and 5 and it can be seen that the olmesartan medoxomil monohydrate is defined by shift of characteristic peaks at about 1705 and 1830 cm⁻¹ of prior-art form for about 20 to 30 cm⁻¹ to the lower wavenumbers and the presence of additional peak at around 1540 cm⁻¹.

Thermogram was obtained with Mettler Toledo DSC822^{e} differential scanning calorimeter. The sample (4-6 mg) was placed in an unsealed aluminium pan with one hole and heated at 5 °C /min in the temperature range from 30 °C to 200 °C in the nitrogen (100 ml/min). Melting points were measured on Koffler microstage

Powder X-ray diffraction spectra (XRPD) of the samples were recorded on Philips PW1710 with reflexion technique: CuKα radiation, range from 2° to 37° 2Theta, step 0.04° 2Theta, integration time 1 sec.

The ultimate form of non orderness of a solid is amorphous state which does not show the repeatability of molecular directions and positions in a solid. Completely amorphous substance thus shows diffuse dispersion of X-ray radiation. The XRPD of a crystalline substance will consist of a series of peaks detected at characteristic scattering angles, which will be substantially independent of the diffractometer used, if the diffractometer is calibrated the values can differ for about 0,05° 2 Theta, taking into account the rounding the differences in values lay in the order of ± 0,1 ° 2Theta, however the different recording conditions or differences in preparing or handling samples can cause the variations from the values reported for as much as ± 0,2 2Theta. The intensities of each specific diffraction peak are a function of various factors, one of those being a particle size and preferred orientation.

For routine work one typically compares the powder pattern of the analyte to that of reference. Identity is established if the scattering angles of the ten strongest reflections obtained for an analyte agree with that of the reference in if the relative intensities of those do not vary by more than 20%. Skilled person will differentiate the form, which is the embodiment of our invention from other forms by comparing the whole X-ray powder diffraction patterns and specifically the strongest peaks, preferably 10, but also any three to five or more distinct peaks (e.g. most intense) for each specific crystalline form.

Following examples further illustrate the invention. They are provided for illustrative purposes only and are not intended to limit in any way the invention.

### EXAMPLES

### EXAMPLE 1 (olmesartan medoxomil hemihydrate) (1668/21)

1 g of olmesartan medoxomil is dissolved in 5 ml of dimethylsulfoxide at room temperature. To a stirred, clear solution 2 ml of water are added and stirring continued for several hours at room temperature. A thick ,crystalline suspension is filtered and dried in vacuo at 50°C. 0.83 g of product obtained in a form of hemihydrate had melting point: 182°C and a characteristic DSC.

### REFERENCE EXAMPLES 1-5

Amorphous and partially crystalline olmesartan medoxomil are prepared according to embodiments described in European patent application publication EP 1 801 111 A1.

### REFERENCE EXAMPLE 1 (amorphous olmesartan medoxomil) (3886)

Clear solution of 1g of olmesartan medoxomil in 100 ml of methanol is vacuum evaporated to dryness at 50°C. Expanded white solid is obtained in yield: 0.97g, melting point 89,5-92,3° C. Amorphous

### REFERENCE EXAMPLE 2 (1667/1)

1g of olmesartan medoxomil is dissolved in 20 ml of ethanol at reflux temperature. Clear solution is vacuum evaporated to dryness at 50°C. Expanded solid is obtained. Yield :1g, melting point 90,5-94,6°C. Amorphous

### REFERENCE EXAMPLE 3 (1667/2)

1g of olmesartan medoxomil is dissolved in 30ml of i-propanol at reflux temperature. Clear solution is vacuum evaporated to dryness at 50°C. Expanded solid is obtained in yield: 1g having melting point:84,6-90,1 °C. Amorphous

### REFERENCE EXAMPLE 4 (1667/3)

Clear solution of 1g of olmesartan medoxomil in 20ml of n-propanol is vacuum evaporated to dryness at 50°C. Expanded solid is obtained in yield: 1g shown by X-ray to be partly crystalline, having degree of crystallinity around 10%.

### REFERENCE EXAMPLE 5 (1668/1)

1g of olmesartan medoxomil is disolved in 30ml of acetonitrile at reflux temperature. Hot solution is filtered and vacuum evaporated to dryness at 50°C. Yield: 1g. Substantially amorphous however partially crystalline, having degree of crystallinity below 5%.

### EXAMPLE 2 (hydrated olmesartan medoxomil)

1 g of substance of Reference Example 1 is exposed to 75% relative humidity at 40°C for one month converts to 1.02 g of hydrated olmesartan medoxomil having crystalline appearance.

### EXAMPLE 3 (pharmaceutical composition)

Substance of previous example is mixed with 27,8 g of lactose and 10 g of microcrystalline cellulose and 3 g of corn starch and blended for short time, whereto 0,3 mg of stearic acid is added. The mass is compressed into tablets and the dissolution tested in 1 M HCl.

## Claims

1. Olmesartan medoxomil hemihydrate.

2. Olmesartan medoxomil hemihydrate according to previous claim **characterized by** that it is crystalline.

3. Olmesartan medoxomil hemihydrate according to previous claim **characterized by** XRPD having strongest diffractions at: 20.6; 22.3; 24,2 ± 0,2° 2Theta.

4. Olmesartan medoxomil hemihydrate according to previous claim **characterized by** XRPD having characteristic pattern exhibiting peaks at 4,0 (40%); 8.0 (10%); 8,7 (50%); 10.4 (50%); 11.9 (30%); 13.5 (40%); 14.3 (40%); 16.0 (20%); 17.0 (40%); 17.5 (40%); 17.9 (20%); 19.6 (10%); 20.1 (70%); 20.6 (90%); 20.9 (70%); 21.0 (80%); 22.3 (100%); 24.2 (100%); 25.8 (30%); 26.2 (20%); 27.9 (30%); 28.2 (40%); 28.9 (40%); 29,3 (30%); 31,2 (20%); 31,5 (20%); 32,4 (10%); °2 Theta, wherein values in parenthesis indicate relative rounded to nearest 10%.

5. Olmesartan medoxomil hemihydrate according to any of the previous claims **characterized by** XRPD having characteristic pattern substantially as in Figure 2.

6. Olmesartan medoxomil hemihydrate according to any of the previous claims **characterized by** endotherm signal showing water removal above 80°C, more considerably above 90°C.

7. A process for manufacturing olmesartan medoxomil in a hydrated form **characterized by** exposure of amorphous form to moisture.

8. The process in accordance with previous claim, wherein the olmesartan medoxomil in a hydrated form is hemihydrate and the exposure is at temperature 40 - 60°C to air with relative humidity between 75% and 95%.

9. A process for manufacturing olmesartan medoxomil hemihydrate **characterized by** isolation from solvent system comprising DMSO and water.

10. The process in accordance with previous claim **characterized by** the weight ratio olmesartan medoxomil : DMSO : water to be 1: 3 - 10 : 1 - 5.

11. The process in accordance with claim 9 or 10 **characterized in that** it consists of following steps:
a) preparing a solution of olmesartan medoxomil in DMSO
b) adding water to the solution of previous step
c) crystallizing and isolating the formed olmesartan medoxomil hemihydrate.

12. The process in accordance with previous claim **characterized by** crystallization and isolation in step c) at room temperature.

13. Use of crystalline olmesartan medoxomil hemihydrate according to claim 1 for preparing a medicament.

14. Olmesartan medoxomil hemihydrate according to claim 1 for use in treatment of hypertension.

15. An immediate release pharmaceutical composition comprising olmesartan medoxomil hemihydrate according to claim 1 and a pharmaceutically acceptable carrier.

## Patentansprüche

1. Olmesartanmedoxomil-Hemihydrat.

2. Olmesartanmedoxomil-Hemihydrat gemäß dem vorangehenden Anspruch, **dadurch gekennzeichnet, dass** es kristallin ist.

3. Olmesartanmedoxomil-Hemihydrat gemäß dem vorangehenden Anspruch, **gekennzeichnet durch** ein Röntgenpulverdiffraktogramm (XRPD), welches die stärksten Diffraktionen aufweist bei: 20,6; 22,3; 24,2 ± 0,2° 2Theta.

4. Olmesartanmedoxomil-Hemihydrat gemäß dem vorangehenden Anspruch, **gekennzeichnet durch** ein ein charakteristisches Muster aufweisendes Röntgenpulverdiffraktogramm (XRPD) mit Peaks bei 4,0 (40%); 8,0 (10%); 8,7 (50%); 10,4 (50%); 11,9 (30%); 13, 5 (40%); 14,3 (40%) ; 16,0 (20%); 17,0 (40%); 17,5 (40%); 17,9 (20%); 19,6 (10%) ; 20,1 (70%); 20,6 (90%) ; 20,9 (70%) ; 21,0 (80%); 22,3 (100%); 24,2 (100%); 25, 8 (30%) ; 26,2 (20%); 27,9 (30%); 28,2 (40%); 28,9 (40%); 29, 3 (30%); 31,2 (20%); 31,5 (20%); 32,4 (10%); °2 Theta, wobei Werte in Klammern Relativwerte gerundet auf die nächstliegenden 10% angeben.

5. Olmesartanmedoxomil-Hemihydrat gemäß irgendeinem der vorangehenden Ansprüche, **gekennzeichnet durch** ein Röntgenpulverdiffraktogramm (XRPD), welches ein charakteristisches Muster im Wesentlichen wie in Figur 2 aufweist.

6. Olmesartanmedoxomil-Hemihydrat gemäß irgendeinem der vorangehenden Ansprüche, **gekennzeichnet durch** ein endothermes Signal, welches oberhalb von 80°C, wesentlicher oberhalb von 90°C, Entwässerung zeigt.

7. Verfahren zur Herstellung von Olmesartanmedoxomil in einer hydratisierten Form, **gekennzeichnet durch** Einwirkung von Feuchtigkeit auf eine amorphe Form.

8. Verfahren gemäß dem vorangehenden Anspruch, wobei das Olmesartanmedoxomil in einer hydratisierten Form Hemihydrat ist und die Einwirkung bei einer Temperatur von 40 bis 60°C durch Luft mit einer relativen Feuchtigkeit zwischen 75% und 95% stattfindet.

9. Verfahren zur Herstellung von Olmesartanmedoxomil-Hemihydrat, **gekennzeichnet durch** Isolierung aus einem DMSO und Wasser umfassenden Lösungsmittelsystem.

10. Verfahren gemäß dem vorangehenden Anspruch, **dadurch gekennzeichnet, dass** das Gewichtsverhältnis von Olmesartanmedoxomil : DMSO : Wasser 1 : 3 - 10 : 1 - 5 ist.

11. Verfahren gemäß Anspruch 9 oder 10, **dadurch gekennzeichnet, dass** es aus den folgenden Schritten besteht:
a) Herstellen einer Lösung von Olmesartanmedoxomil in DMSO
b) Zugeben von Wasser zur Lösung des vorangehenden Schritts
c) Kristallisieren und Isolieren des gebildeten Olmesartanmedoxomil-Hemihydrats.

12. Verfahren gemäß dem vorangehenden Anspruch, **gekennzeichnet durch** Kristallisation und Isolierung in Schritt c) bei Raumtemperatur.

13. Verwendung von kristallinem Olmesartanmedoxomil-Hemihydrat gemäß Anspruch 1 zur Herstellung eines Medikaments.

14. Olmesartanmedoxomil-Hemihydrat gemäß Anspruch 1 zur Verwendung in der Behandlung von Bluthochdruck.

15. Pharmazeutische Zusammensetzung mit unmittelbarer Freisetzung, welche Olmesartanmedoxomil-Hemihydrat gemäß Anspruch 1 und einen pharmazeutisch annehmbaren Träger umfasst.

## Revendications

1. Olmésartan médoxomil semi-hydraté.

2. Olmésartan médoxomil semi-hydraté selon la revendication précédente **caractérisé par le fait qu'**il est cristallin.

3. Olmésartan médoxomil semi-hydraté selon la revendication précédente **caractérisé par** une diffractométrie de poudre par rayon X (XRPD) présentant les diffractions les plus puissantes sur 2 thêta à : 20,6 ; 22,3 ; 24,2 ± 0,2°.

4. Olmésartan médoxomil semi-hydraté selon la revendication précédente, **caractérisé par** une diffractométrie XRPD présentant un profil caractéristique avec des pics à 4,0 (40%) ; 8,0 (10%) ; 8,7 (50%) ; 10,4 (50%) ; 11,9 (30%) ; 13,5 (40%) ; 14,3 (40%) ; 16,0 (20%) ; 17,0 (40%) ; 17,5 (40%) ; 17,9 (20%) ; 19,6 (10%) ; 20,1 (70%) ; 20,6 (90%) ; 20,9 (70%) ; 21,0 (80%) ; 22,3 (100%) ; 24,2 (100%) ; 25,8 (30%) ; 26,2 (20%) ; 27,9 (30%) ; 28,2 (40%) ; 28,9 (40%) ; 29,3 (30%) ; 31,2 (20%) ; 31,5 (20%) ; 32,4 (10%) ; sur 2 thêta, dans lequel les valeurs entre parenthèses indiquent la valeur relative arrondie aux 10% les plus proches.

5. Olmésartan médoxomil semi-hydraté selon l'une quelconque des revendications précédentes, **caractérisé par** une diffractométrie XRPD présentant un profil caractéristique sensiblement identique à celui de la figure 2.

6. Olmésartan médoxomil semi-hydraté selon l'une quelconque des revendications précédentes, **caractérisé par** un signal endotherme montrant une élimination d'eau au-dessus de 80°C, de manière plus considérable au-dessus de 90°C.

7. Procédé de fabrication d'olmésartan médoxomil sous une forme hydratée **caractérisé par** l'exposition d'une forme amorphe à de l'humidité.

8. Procédé selon la revendication précédente, dans lequel l'olmésartan médoxomil sous une forme hydratée est semi-hydraté, et l'exposition se fait à une température de 40 à 60°C, à l'air présentant une humidité relative comprise entre 75% et 95%.

9. Procédé de fabrication d'olmésartan médoxomil semi-hydraté **caractérisé par** l'isolation à partir d'un système de solvant comprenant du DMSO et de l'eau.

10. Procédé selon la revendication précédente **caractérisé par** un rapport en poids de olmésartan médoxomil : DMSO : eau dans la plage de 1 : 3 à 10 : 1 à 5.

11. Procédé selon la revendication 9 ou 10, **caractérisé en ce qu'**il comprend les étapes suivantes :
a) de préparation d'une solution d'olmésartan médoxomil dans du DMSO,
b) d'ajout d'eau à la solution de l'étape précédente,
c) de cristallisation et d'isolation de l'olmésartan médoxomil semi-hydraté formé.

12. Procédé selon la revendication précédente, **caractérisé par** la cristallisation et l'isolation de l'étape c) à température ambiante.

13. Utilisation d'olmésartan médoxomil semi-hydraté cristallin selon la revendication 1 afin de préparer un médicament.

14. Olmésartan médoxomil semi-hydraté selon la revendication 1, en vue d'une utilisation pour le traitement de l'hypertension.

15. Composition pharmaceutique à libération immédiate comprenant l'olmésartan médoxomil semi-hydraté selon la revendication 1 et un excipent pharmaceutiquement compatible.
